(19) Europäisches Patentamt
European Patent Office
Office européen des brevets

(11) Publication number: **0 100 168 B1**

(12) **EUROPEAN PATENT SPECIFICATION**

(45) Date of publication of patent specification: **28.12.88**

(51) Int. Cl.[4]: **A 61 K 9/16,** A 61 K 9/20

(21) Application number: **83303935.7**

(22) Date of filing: **06.07.83**

(54) **Processes for preparing tablets by a modified 'wet-granulation' technique.**

(30) Priority: **06.07.82 GB 8219487**

(43) Date of publication of application:
**08.02.84 Bulletin 84/06**

(45) Publication of the grant of the patent:
**28.12.88 Bulletin 88/52**

(84) Designated Contracting States:
**AT BE CH DE FR IT LI LU NL SE**

(56) References cited:
**GB-A-1 287 431**
**GB-A-1 370 479**
**GB-A-1 410 909**
**US-A-3 873 694**

**ULLMANN'S ENZYKLOPÄDIE DER TECHNISCHEN CHEMIE, 4th edition, vol. 18, 1979 VERLAG CHEMIE, Weinheim**

(73) Proprietor: **STERWIN AG.**
**Zeughausgasse 9**
**CH-6300 Zug (CH)**

(72) Inventor: **Rogerson, Alan George**
**16 Heathdale Gardens**
**High Heaton Newcastle Tyne and Wear (GB)**

(74) Representative: **Green, Alan James et al**
**SANDERSON & CO. European Patent Attorneys**
**34, East Stockwell Street**
**Colchester Essex CO1 1ST (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 100 168 B1

**Description**

This invention relates to processes for preparing tablets by a modified "wet-granulation" technique—and especially, but not exclusively, tablets which have a high active-matter content.

A variety of substances meant to be taken by humans and other animals (especially, but not exclusively, pharmaceutical substances intended for oral administration) are often formulated as tablets.

The term "tablet" should be sufficiently familiar to require no explanation; but, in case of need, can be defined as follows:

as used herein the term "tablet" includes not only tablets proper but also similar discete bodies, perhaps of other shapes and sometimes known by different names, above all so-called "caplets" (i.e. capsule-shaped tablets, which are easier than tablets to swallow) and also such things as lozenges, pills, troches and drageens—and the term is here used to refer to mixtures of particulate solid materials, which have been brought together in various ways and finally compressed so that they become compacted into shaped entities able to persist under normal handling conditions but to disintegrate at the desired site, usually within the body and above all in the digestive tract.

Most tablets are intended to be swallowed, and thus must be kept within the maximum bulk-limit which (dependent slightly upon its shape) an average person is able and willing to swallow.

The "size" of the tablets they produce is conventionally defined by tabletters in terms not of their bulk but of their weight. There is room for debate as to what precisely is the absolutely maximum "swallowable" size of tablet, which must depend on the shape of the tablet and on the individual who is to swallow it. We however take the view that the absolutely maximum "swallowable" size of tablet can be set at a weight of no more than say 1200 milligrams; and many might choose to set it much lower at a weight of not more than say about 850 milligrams. Whatever weight limit one adopts (be it 850 mg or 1200 mg, or preferably something in between—usually say no more than 1 g) it follows that any tablet which is to be swallowed must accommodate all of its ingredients, not only the active-matter but also every other necessary or desirable type of ingredient, within that weight limit. Moreover, that consideration not only applies to tablets intended to be swallowed, but to some extent also affects tablets of other kinds, because so much of the available tabletting machinery is dimensioned to produce swallowable tablets that in practice it is often most convenient to make other kinds of tablets on the same machinery and thus to the same dimensions.

For most purposes all necessary ingredients can be accommodated within an 850 mg tablet, and certainly within a 1200 mg tablet. There are, however, situations in which the above-discussed overall weight limitations on tablet size create a hitherto-insurmountable obstacle.

In a pharmaceutical context, one can readily appreciate that the dose necessary at any one administration should desirably be given in the fewest possible tablets, thus if possible in just a single tablet. In the relatively rare event that the dose of active-matter necessary at any one administration should exceed the maximum swallowable size of tablet, clearly it is then quite impossible for that dose to be contained in just one swallowable tablet. More usually, however, the amount of active-matter to be given at any one administration is less than the swallowable maximum, so that notionally it could all be contained within a single tablet—and yet in practice that till now has proved impossible with some compounds and combinations of compounds, because the amount of active-matter so closely approaches the swallowable maximum that the balance is not enough to accommodate the tabletting aids (and possibly other ingredients) which are pharmacologically-inert but whose presence is vital to the manufacture of a satisfactory tablet when, as so often, the compression characteristics of the active-matter are poor.

In the manufacture of tablets, the final compression of each tablet takes place between the punches within a die, after the latter has been filled with the desired mixture of particulate solid materials. Normally such a mixture is one which has been pregranulated and such differences as exist between conventional tabletting techniques lie primarily in the respective procedures used for preliminary processing of the mixture of particulate solid materials before they enter the die. Thus, when all the ingredients (including the active-matter) have good compression characteristics, one may be lucky enough to be able to adopt the simplest and cheapest of techniques known as "dry granulation", or a modified version thereof involving what is called "preliminary slugging". All too often, however, the compression characteristics of the mixture are so poor (a defect attributable usually to the nature and/or amount of active-matter present) that one is driven back as a last resort on the technique known as "wet-granulation".

There is much art and skill in practising the "wet-granulation" technique but, in outline, it involves no more than the incorporation of a granulating fluid into the mixed, powdery tablet ingredients (including at least some tabletting aids) in such an amount and manner as to convert them into a uniform, moist, coherent, non-pasty mass—which is at least essentially non-flowing—and which then is formed into moist granules of fairly uniform size, usually by forcing the mass through a screen. Thereafter the moist granules are dried and re-screened to break down agglomerates, and finally blended with other tabletting aids so as thus to arrive at the granulate ready for tabletting.

It will be noted that in "wet-granulation" the tablet ingredients besides the active-matter also conventionally include other, pharmacologically-inert materials, certainly tabletting aids and perhaps also bulking agents. Some of such tabletting aids may be included in the mixed, powdery ingredients before the granulating fluid is incorporated therein, while further tabletting aids may be applied to the surfaces of the

granules, and in between them, after the granules have been formed and before the granulate is passed to the tabletting machine.

At this point it should be observed that when the amount of active-matter per tablet is small the pharmacologically-inert materials conventionally might well include what are here described as bulking agents, that is to say filler-type materials which serve no function except to bulk out the mixture so as to make tablets of adequate size; but that is not the kind of situation with which this invention is primarily concerned, and thus mere bulking agents are not intended to be included in the term "tabletting aids" as used herein.

In the kind of situation in which one resorts to a "wet-granulation" technique, thus when the nature and/or amount of the active-matter causes the mixture to have poor compression characteristics, it will however usually be necessary to incorporate some appropriate amount of some or all of the conventional types of tabletting aids, namely binders, glidants, lubricants and disintegrants. Broadly-speaking:

binders are substances which help bind the particles of powder together in a form suited to compaction and compression;

glidants are substances which aid filling of the particles and/or granules into the die before compression;

lubricants are substances which help the compressed tablets to leave the die; and

disintegrants are substances which help the tablet to disintegrate, and perhaps dissolve, when it reaches its ultimate destination, usually within the body.

Quite a variety of materials is available to serve each of these functions, some of them more effective than others. Sometimes a given material may be capable of simultaneously performing more than one function and, of course, all of them cannot help but bulk out the tabletting mixture, even though perhaps that may not be desired. It will, therefore, be appreciated that one cannot make any wholly-reliable predictions about the absolute and relative amounts of each individual binder and/or glidant and/or lubricant and/or disintegrant which should be present, in a successful formulation made by "wet-granulation" one can however as a generalization say that the overall requirement for all four types of tabletting aids will nearly always fall in the range of from 5% to 25% w/w, calculated relative to the weight of the final (dry) powder mass, and indeed the overall percentage of tabletting aids usually need not exceed say 15% w/w.

Even using the conventional "wet-granulation" technique it is however not always possible to achieve a "successful" formulation, that is to say one which on compression of the granulate yields tablets which conform to accepted standards of appearance, hardness, fragility (or friability), disintegration and uniformity of weight, unless indeed the above-indicated overall percentage of tabletting aids is still further increased. The problem does not usually lie with the tabletting aids themselves, for these can be chosen at will from the well-tried array of such materials introduced over very many years, and can be relied upon to serve their function admirably, to the extent that they are not prevented from so doing by the nature of the active-matter. It is indeed the active-matter which is liable to cause tabletting problems, for here there is no freedom of choice—the tabletter must seek to incorporate in his tablet whatever active-matter the clinician wants—if possible in an amount which represents a unit dose suitable at one administration. While some kinds of active-matter, at least when present in moderate proportions, will lend themselves readily to compression and compaction into good-quality tablets when accompanied only by conventional proportions of tabletting aids, others will not. Furthermore, as new kinds of active-matter are introduced which have poor compression characteristics and/or as the clinician seeks to prescribe higher dosage levels of existing kinds of "poorly-compressible" active-matter, the tabletter is faced with fresh problems, sometimes very difficult or indeed impossible to solve within the confines of a single tablet. Where high-dosage active-matter (or mixtures thereof) with poor compression characteristics must be incorporated into a single tablet, even conventional wet-granulation techniques using normal levels of tabletting aids prove inadequate to resolve these problems; yet if one uses still higher levels of tabletting aids, that may well result in a mixture of such bulk volume that the required weight is difficult or impossible to accommodate within the compression die cavity. However, even if the mixture can be so accommodated and tablets compressed, the tablets then may be of such a large size that they are difficult to swallow.

It is the problem of tabletting such poorly-compressible kinds of active-matter, and especially those which are clinically-prescribed at high-dosage levels, which we have tackled. In our investigation of that problem we have been led to question hitherto-prevailing assumptions concerning the procedures employed in the formation of tablets, with the aim of finding some way of making good-quality tablets even from poorly-compressible kinds of active-matter which tablets however contain no more than the unavoidable proportion of tabletting aid(s) necessarily present in "successful" conventional formulations based on easily-compressible active matter.

GB—A—1,287,431 relates to improvements in pharmaceutical formulations and, in particular, to a process for preparing coated particles of paracetamol.

GB—A—1,410,909 relates to pharmaceutical compositions. The compositions are ones containing paracetamol and a method is provided of preparing a composition containing paracetamol which is suitable for direct tablet compression.

We have now found surprisingly that by employing a modified version of conventional "wet-granulation", which for convenience we term "slurry granulation", we can provide a process for

manufacturing tablets which goes at least some way to achieve the much-desired goal mentioned above before the acknowledgement of GB—A—1,287,431 and GB—A—1,410,909.

Accordingly, the present invention provides a process for preparing a granulate suitable for use in manufacturing tablets by compressing and compacting a said granulate, which process comprises forming an intimate mixture of particulate-solid materials including active-matter and tabletting aids, as well as optionally other pharmacologically-inert material(s), thereafter "wet-granulating" said mixture by moistening it with substantially non-solvent (as hereinafter defined) granulating fluid(s) to form a substantially-uniform, moist, coherent, non-pasty mass which finally is granulated (i.e. subdivided into individual granules) and dried, the dried granules (optionally after adding further tabletting aids thereto) constituting a granulate ready for compression and compaction into tablets, the process being characterised by including the steps of:

(A) predetermining a quantity of granulating fluid(s) needed to convert the entire particulate-solid mixture into a desired moist, coherent, non-pasty mass;

(B) homogenizing part only of the particulate-solid material(s), separately from the remainder thereof, with a chosen amount of granulating fluid(s), that chosen amount being at least 90% by weight of the aforesaid predetermined quantity of granulating fluid(s), so as to form a substantially-homogeneous slurry wherein the percentage by weight of solids in the slurry, namely:

$$\frac{\text{Total Solids, both dissolved and undissolved} \times 100}{\text{Total Slurry, i.e. fluids plus total solids}}$$

is at least about 25% w/w, preferably at least about 30% w/w; and

(C) moistening the remaining part of the particulate-solid material(s) in the manner of "wet-granulation" but with the slurry resultant from step (B) above (and thereafter as necessary with the balance of the granulating fluid(s), if any) so as thus to form a desired, substantially-uniform, moist, coherent, non-pasty mass ready for granulation.

It will be seen that the process of this invention involves separately homogenizing part of the particulate-solid material(s) with the granulating fluid(s) to form a substantially-homogeneous slurry which is then used to moisten the remainder otherwise in the manner of conventional "wet-granulation". While it is at present impossible for us definitely to say why this technique should enable lesser proportions of tabletting aids to be satisfactorily employed than would be needed in a fully-conventional "wet-granulation", we are inclined to think that homogenization of part of the ingredients must somehow contribute to an "improvement" of the compression-characteristics of the material in the slurry. It is reasonable to suppose that the overall compression-characteristics of any granulate must partly depend upon the compression-characteristics of each of its ingredients, but which may operate via more than one mechanism. Poorly-compressible materials quite often have relatively fine particle sizes, and thus form low bulk-density "flocculant" powders, wherein a proportion of the particles are aggregated into multi-particle clumps. Therefore, one may speculate that homogenization perhaps tends to reduce the proportion of multi-particle clumps, and/or to ensure that substantially all of the particles are properly wetted with the granulating fluid. These, however, are only suppositions, and we do not desire to be limited to any theoretical considerations, preferring in the present uncertain state of our knowledge simply to observe that our new "slurry-granulation" process works in situations where the conventional "wet-granulation" process does not.

It is convenient here to observe the term "active-matter" is used throughout this specification in a broad sense which encompasses whatever the manufacturer may wish to characterize his tablets, thus for instance including either sweetening and flavouring agents in the case of confectionary products or dietary aids both of the assimilable and of the non-assimilable kind. The invention, however, is primarily concerned with active-matter which consists of one or more pharmacologically-active substances.

The predetermined "total" amount of granulating fluid to be employed (either in the granulating slurry as such, or otherwise) obviously must not exceed that needed to achieve the desired moist, coherent but non-pasty mass. When that amount (which, as known to skilled tabletters, is parly dependent on the type and scale of equipment used, as well as upon the degree of mechanical working imposed upon the mix during its preparation and subsequent screening) is exceeded, then the mixture will clog during screening to form the moist granules. Equally, however, the predetermined "total" amount of granulating fluid to be employed cannot be less than the amount needed to achieve the desired moist, coherent, non-pasty mass.

Predetermination of the total quantity of granulating fluid(s) needed to convert the entire particulate-solid mixture into a desired substantially-uniform, moist, coherent, non-pasty mass can and indeed initially will be achieved empirically, as is always the case in conventional procedures. A skilled operative is usually able to make an intelligent guess at the approximate quantity of granulating fluid(s) which will be required; but the exact amount is found by trial, adding a little at a time until the desired effect is achieved. Of course, the proper quantity of granulating fluid(s) having once been determined for a certain mixture of ingredients it will not always be necessary to redetermine it subsequently. Repetition of the process in the same manner on the same batch-size of the same ingredients will require essentially the same, already predetermined quantities of granulating fluid(s). In such situations step (1) of the process of this invention

becomes notional rather than actual—but, even if only notionally, it does for all that form part of the process.

In passing it should be noted that the quantity of granulating fluid(s) required, will of course be dependent, as in conventional "wet-granulation", upon the nature of the ingredients in the powdery mixture to be granulated, upon the parameters of the mixing procedure employed, and upon the kind of moist, coherent, non-pasty mass required. As general guidance it can be said that the total amount of granulating fluid(s) predetermined for use according to the present invention, although not necessarily exactly identical, will nevertheless be much the same as the amount needed to moisten the same mass using the conventional "wet-granulation" technique. However, the exact amount of granulating fluid to be employed is a matter which can and indeed must ultimately be left, as in conventional wet-granulation techniques, to the skill and judgement of the operative seeking to achieve the desired characteristics in the granulate and tablets compressed therefrom.

The homogenization of the particulate-solid material(s) with the granulating fluid(s) must, as indicated above, be carried out in such a way as to form a substantially-homogeneous slurry, that is to say a flowable suspension (having the specified, relatively high percentage w/w of solids) which shows no obvious externally-visible sign of segregation over the longest period encountered as an acceptable delay in manufacturing procedures, thus in the course of a few hours, preferably not more than 2 hours. In everyday terms, one seeks a suspension with something akin to the consistency and stability of cream. That kind of suspension may be achieved in any convenient manner, but in our experience is best prepared by means of a high-shear homogenizer.

We do not exclude the possibility that the part of the particulate-solid material(s) which is to be slurried with granulating fluid(s) can be any part thereof, thus some or all of any of the individual ingredients, or even just some of the entire unsegregated mixture. However, we have found empirically that the best results seem to be obtained when the material which is slurried is at least mostly active-matter. This ties in with our tentative supposition that homogenization somehow "improves" the compression- characteristics of the material being slurried—because, as previously explained, the tabletting aids have already been selected for their beneficial effect upon the compression-characteristics of the granulate and need little improvement in this respect, but the compression-characteristics of the active-matter often leave much to be desired.

As a rule of thumb it seems to be the case that the part of the particulate-solid materials which is homogenized with the chosen amount of granulating fluid(s) should contain not more than 50% w/w, and usually in our experience not more than about 35% w/w, of the total active matter; but, on the other hand, we have found that generally-speaking the slurry should contain not less than 10% w/w and usually not less than about 15% w/w, of the total active matter.

Taking a different approach, it also seems to be the case that the part of the particulate-solid materials which is homogenized wihh the chosen amount of granulating fluid(s) usually need not exceed 50% w/w of the total particulate-solid materials, and generally-speaking need not be more than about 35% w/w; but, on the other hand, usually will not be less than 10% w/w, and often not less than about 15% w/w, of the total particulate-solid material.

At this point it is appropriate to observe that the slurry granulation process of this invention naturally can be employed in the tabletting of any kind of active-matter, even easily-compressible kinds of active-matter. This slurry granulation process however has been developed by us with the particular objective of improving the tabletting of "poorly-compressible" active-matter, and it is there that it displays itself to best advantage. Regrettably, there is no universally-accepted definition or test which can be used to identify "poorly-compressible" material, but any experienced tabletter will intuitively recognize what we mean thereby.

It is also appropriate here to observe that there is nothing which forbids the use of the slurry granulation process of this invention in the preparation of tablets containing only relatively low proportions of active-matter—but, again, the process has been developed by us with the particular objective of facilitating the preparation of tablets containing higher-than-usual proportions of active matter, and it is in that area that the process shows itself in the most advantageous light. By "higher-than-usual" proportions of active matter we choose, somewhat arbitrarily, to mean tabletting mixtures and tablets made therefrom wherein the proportion of active matter relative to the whole (dry) mixture is 80% w/w or more.

While we do not go so far as to claim that it can always be achieved, we have found that using our slurry granulation process it is generally possible to form satisfactory tablets even from materials which are poorly-compressible and/or present in the higher-than-usual proportions while utilizing the levels of tabletting aids which would conventionally be employed in "wet-granulation" yet which would fail to be sufficient with such materials were it not for the slurry granulation technique of this invention.

Thus, with such materials the present process can employ from 5% to 25% w/w, and often only from 5% to 15% w/w, of tabletting aids overall; and within those overall ranges some or all of the following:

binders in the range of from 2% to 10% w/w;
glidants in the range of from 0.2% to 10% w/w;
lubricants in the range of from 0.2% to 4% w/w; and
disintegrants in the range of from 2% to 10% w/w.

Clearly, the process of the invention displays its advantages most outstandingly when the active

matter is poorly-compressible and needs (at least for some clinical purposes) to be present in higher-than-usual proportions. Examples of active matters which fall in this dual-problem category are for instance:

(a) so-called nalidixic acid, that is to say 1 - ethyl - 1,4 - dihydro - 7 - methyl - 4 - oxo - 1,8 - naphthyridine - 3 - carboxylic acid;

(b) so-called paracetamol, that is to say N - (4 - hydroxyphenyl) - acetamide, with or without, but especially with so-called methionine, that is to say 2 - amino - 4 - (methylthiol) - butyric acid;

(c) so-called hexopal, that is to say *myo* - inositol hexa - 3 - pyridine - carboxylate;

(d) so-called benorylate, that is to say 2 - (acetyloxy) benzoic acid 4 - (acetylamino) phenyl ester, and

(e) so-called paracetamol methionate, that is to say N - acetyl - *para* - aminophenyl - N' - acetyl - D,L - methionate, either alone or in admixture with paracetamol.

Besides the immediately obvious candidates for the slurry-granulation process of this invention which have been mentioned above there are of course others which suggest themselves such as the sulphonamides.

The chosen amount of granulating fluid(s) used to form the slurry will be in the range of from 90% up to 100% by weight of the previously predetermined total quantity already discussed above. If there is any uncertainty about quite how much granulating fluid will be needed, then a little can be held back (thus not included in the slurry) to be added at discretion after the slurry has been incorporated into the mix. Except in that situation it is however preferred that the "chosen amount" should be substantially the same as the "predetermined-quantity") because, for reasons which we do not at present fully understand, it does seem that in order to secure the maximum improvement in the compression characteristics of the final granulate it is desirable that the chosen amount of granulating fluid used in formation of the slurry should be substantially all of the predetermined quantity, so that the resultant slurry alone will be substantially sufficient to achieve the desired moist, coherent but non-pasty mass containing all of the necessary ingredients, without the need for any significant further addition of granulating fluid. Thus, it is preferred to use all or virtually all of the granulating fluid(s) to form the slurry.

The lower end of the weight percentage of solids in the slurry, set at a minimum of about 25% w/w, is necessary to ensure that a sufficient proportion of the total particulate-solid material(s) undergoes the slurry in step. The upper end of the weight percentage of solids has no theoretical maximum, but is limited by the increasing practical difficulties of forming the slurry and of using it to moisten the particulate-solids mix as the percentage w/w of solids rises. Thus, genreally one forms a slurry with as high a percentage w/w of solids as can conveniently be achieved and utilized. Usually, the percentage w/w of solids in the slurry will not exceed 80% w/w, and in our experience so far we have never significantly exceeded about 70%. Typically, we have found it both readily possible and entirely satisfactory to form and use a slurry having a percentage w/w of solids in the range of from 40% up to 60% w/w; and we prefer to aim at a percentage w/w of solids of around 50% w/w, thus say 50±5% by weight.

The granulating fluid(s) used in the process of this invention to form the slurry and thereafter to moisten the mixture must, as previously indicated, be substantially non-solvent for the overwhelming proportion (at least 90% w/w, and usually 95% w/w or more) of the particulate-solid materials to be moistened thereby. That requirement however does not necessarily mean that the granulating fluid(s) used in the process must be inherently non-solvent for all of the ingredients. On the contrary, it is indeed a possible and sometimes preferred feature of the process of this invention that the non-solvent granulating fluid should be or should include a liquid which inherently is a solvent for some of the ingredient(s), provided only that the inherently-solvent fluid is rendered substantially non-solvent by substantially saturating or even super-saturating it with that solute.

Nevertheless it is in fact often quite desirable that the otherwise non-solvent granulating fluid should serve as a solvent for a minor proportion of the tabletting aid(s). Specifically, we have found it usually is advantageous if at least part of the binding agent, and normally all of it, is dissolved in the granulating fluid(s) incorporated in the granulating slurry.

Examples of such conventional binding agents include starch, soluble starch, gelatin, polyvinylpyrrolidone and/or cellulose derivative(s). Of all the various binding agents we have so far employed the best by far, possibly because of its wide-ranging solubility profile in readily-available liquids, has proved to be polyvinylpyrrolidone (PVP), which therefore we much prefer to employ—and desirably to dissolve in the continuous phase of the granulating slurry.

It will of course be appreciated that, as in conventional practice, the granulating fluid(s) employed to form the granulating slurry can sometimes advantageously consist of or include a substantially non-toxic, organic liquid more volatile than water, and in that event the process should be carried out if appropriate under flame-proof conditions. In those cases where an ingredient or ingredients partly dissolve in the granulating fluid(s), it may be desirable or even sometimes preferred to include a crystal poison in the mixture which is processed. Thus, for example, lactose or more preferably sodium acetate may be included as a crystal poison.

Dependent upon the nature of the various particulate-solid ingredients and other operating parameters, the granulating fluid(s) employed can be any of these used in conventional wet-granulation techniques. Thus, for instance, the granulating fluid can consist of water alone or it can include water; and/or it can consist of or include any one or more water-miscible relatively-volatile polar organic liquids

for example, methanol, ethanol, *iso*propanol, acetone and/or methyl ethyl ketone; and/or it can consist of or include any one or more water-immiscible, relatively-volatile organic liquids, for example, above all methylene chloride but also perhaps (subject to operational difficulties and dangers) chloroform or ether.

When seeking to achieve one of the primary objectives of this invention, namely the preparation of tablets containing higher-than-usual proportions of active-matter, no more bulking agents will be employed. There is, however, no other objection to the incorporation of bulking agents in the particulate-solid materials used in the process of this invention. Supposing that any such bulking agents are to be included those selected may of course be any of the conventional ones, chosen in accordance with prevailing practice, and thus will probably consist of or include one or more of the following, namely lactose, calcium phosphate, mannitol starch and/or soluble starch.

After the remaining part of the particulate-solid material(s) has been uniformly moistened by means of the granulating slurry, the resultant moist, coherent, non-pasty mass is broken down into moist granules in any conventional manner, usually by screening.

The moist granules must than be dried. Drying can be effected by any convenient method, and various conventional drying techniques are available. We would ourselves suggest drying, for example, either in loosely-packed drying trays, or more conveniently in a fluidised-bed dryer.

The process of this invention will normally include the step of blending the tabletting granules with one or more further tabletting aid(s) before compacting the granulate to form tablets in conventional tabletting machinery. These "post-granulation" tabletting aid(s) will usually consist of or include disintegrant(s) and/or lubricant(s). Such disintegrant(s) may consist of or include a component or mixture of components capable of imparting an effervescent character to the final tablet, but in that event all the parameters of the process must of course be chosen so as to form the final tablet without detriment to its desired effervescent character.

This invention of course also extends to tabletting granulates and also tablets made therefrom whenever prepared in or by the process herein disclosed.

In addition, the invention also provides a tablet of overall weight no more than 1 g, which tablet comprises a unit dose of active matter of at least 700 mg, the active matter being paracetamol together with methionine or paracetamol methionate, or paracetamol methionate alone, present in an amount of from 80 to 90% by weight, and the tablet having a hardness of from 7 to 15 kp, a friability of less than 2%, preferably less than 1% and affording a disintegration time of no more than 15 minutes, preferably from 5 to 15 minutes.

In order that the invention may be more fully understood in all of its various aspects it will now be described in more detail, though only by way of illustration, with reference to the following specific Examples:—

Example 1

Preparation of tablets containing paracetamol and DL-methionine using wholly-aqueous granulating slurry

Stage A: Preparation of tabletting mixture

A tabletting mixture was formed by mixing together the ingredients which are listed in Table I below, in the absolute and proportionate amounts also shown in Table I, by means of the procedure described subsequently herein:

TABLE I

| Ingredients | Total quantity in grams | Amount in slurry |
|---|---|---|
| Active-matter | | |
| DL-Methionine | 250 | 125 |
| Paracetamol | 500 | — |
| (Sub-Total of Active Matter) | (750) | (125) |
| Tabletting aids | | |
| Polyvinylpyrrolidone | 30 | 30 |
| Stearic acid | 10 | — |
| Sodium starch glycollate | 50 | — |
| (Sub-Total of Tabletting Aids) | (90) | (30) |
| Total: | 840 | 155 |
| Granulating Fluid | Water:200 mls | 200 |

Stage B: Preparation of the granulate

The procedure used for the preparation of the granulate from the above-listed ingredients was as follows:

Preparation of the slurry

The polyvinylpyrrolidone (30 grams) was dissolved in 200 mls of water. Half of the DL-methionine (i.e. 125 grams) was suspended therein by means of a high-shear homogenizer to form a stable dispersion having a cream-like consistency, which was used as the granulating slurry in the manner described below.

Preparation of the moist mass:

The remainder of the DL-methionine (i.e. 125 grams) was mixed with the paracetamol (500 grams) and half the sodium starch glycolate (25 grams) in a conventional mixer-granulator. The mixture was then wet-granulated until suitably massed in an otherwise conventional manner except that as granulating fluid there was used the granulating slurry prepared as described in Stage A above.

Preparation of the granules:

The resultant moist, coherent, non-pasty granulate was screened through a 4-mesh sieve, and dried in a fluidized-bed drier at a temperature of from about 50° to 60°C until a moisture-content of less than 2% had been achieved.

The resulting dried granules were re-screened through a 16-mesh sieve to eliminate agglomerates and then blended with stearic acid (10 grams) and the remainder of the sodium starch glycolate (25 grams) to form the final granulate ready for tabletting.

Stage C: Preparation of tablets

The free-flowing tabletting granulate prepared as described in Stage B above was fed into a suitable conventional tablet-press, and compacted thereby into tablets which had an overall weight of approximately 840 mg each. Despite the fact that paracetamol is poorly-compressible and DL-methionine still more so, yet these were together present to an extent of nearly 90% w/w, with only about 10.7% of tabletting aids and no mere bulking agents. Furthermore, these tablets were found to conform to accepted standards of appearance, hardness, fragility (friability), disintegration and uniformity of weight.

0—0—0—0—0—0—0—0—

Example 2

Preparation of tablets containing paracetamol and methionine, using non-aqueous or semi-aqueous granulating fluids

Stage A: Choice of ingredients of mixture

A tabletting mixture was formed, by the procedure described below, from the ingredients listed together with their absolute and proportionate amounts in Table II as follows:

TABLE II

| Ingredients | Quantity in mixture (grams) | Amount in slurry (grams) |
|---|---|---|
| Active matter | | |
| DL-Methionine | 250 | 125 |
| Paracetamol | 500 | — |
| (Sub-Total of Active Matter) | (750) | (125) |
| Tabletting aids: | | |
| Sodium starch glycolate | 50 | — |
| Stearic acid | 10 | — |
| Polyvinylpyrrolidone | 30 | 30 |
| (Sub-Total of Tabletting Aids) | (90) | (30) |
| Totals: | 840 | 155 |
| Granulating Fluid — Water and various *iso*propanol/ Water mixtures | 200 mls | 200 (water) |

Stage B[1]: Preparation of comparative granulate by conventional 'wet granulation'

A preliminary run, using the above-listed ingredients in a standard mixer-granulator, established that approximately 200 mls of water (containing the polyvinylpyrrolidone) added as granulating fluid in the conventional manner were needed to obtain the desired moist, coherent, non-pasty mass suitable for granulation.

The mass was then granulated by screening and drying in a fully conventional manner.

Stage B[2]: Preparation of granulate by 'slurry granulation' in accordance with the present invention

Using a standard mixer-granulator, the above-listed ingredients were brought together in the following manner:

Preparation of the slurry:

The polyvinylpyrrolidone (30 grams) was dissolved in the predetermined amount, namely 200 mls, of one of the following granulating fluid systems:

(a) *iso*propanol:
(b) a mixture (80:20 v/v) of *iso*propanol and water:
(c) a mixture (60:40 v/v) of *iso*propanol and water:
(d) a mixture (40:60 v/v) of *iso*propanol and water: and
(e) a mixture (20:80 v/v) of *iso*propanol and water.

Dissolution was carried out at room temperature under flame-proof conditions, since the flashpoint of *iso*propanol is only 55°F (about 13°C).

Under the same conditions, half of the DL-methionine (i.e. 125 grams) was suspended therein by means of a high-shear homogenizer to form a stable dispersion having a cream-like consistency, which was used in the manner described below as a granulating slurry in the formation of granulate.

Preparation of the moist mass:

The remainder of the DL-methionine (125 grams) was mixed with all the paracetamol (500 grams) and half of the sodium starch glycolate (25 grams) in a conventional mixer-granulator. The dry mixture was then wet-granulated until suitably massed in an otherwise conventional manner except that as the granulating fluid for that purpose there was used the granulating slurry prepared as described above.

Preparation of the granules:

The resultant moist, coherent, non-pasty granulate was screened through a 4-mesh sieve, and dried in

a fluidized-bed air-drier at a temperature of about 40°C for a period of about 20 minutes until the odour of *iso*propanol had disappeared.

The resultant dried granules were re-screened through a 16-mesh sieve to eliminate agglomerates and then blended with the stearic acid (10 grams) and the remainder of the sodium starch glycollate (25 grams) so as to form the final granulate.

Stage C$^1$: Preparation of comparative tablets

The granulate prepared as described in Stage B$^1$ above was compressed into approximately 840 mg tablets upon a suitable conventional rotary tabletting machine. The resultant tablets were however unsatisfactory, due to major problems with the flow of the granulate into the dies and of lamination or splitting of the compressed tablets.

Stage C$^2$: Preparation of tablets according to this invention

The granulate prepared as described in Stage B$^2$ above was fed to the same conventional rotary tabletting machine, and there compressed to form approximately 840 mg tablets. These were found to conform to accepted standards of appearance, hardness, fragility (friability), disintegration and uniformity of weight.

—0—0—0—0—0—0—

Example 3

Preparation of tablets containing both paracetamol and paracetamol methionate using methanol as the granulating fluid

Stage A: Preparation of tabletting mixture

A tabletting mixture was formed by mixing together the ingredients which are listed in Table III below, in the absolute and proportionate amounts also shown in Table III, by means of the procedure described subsequently herein:

TABLE III

| Ingredients | Quantity in whole mixture (grams) | Amount in slurry (grams) |
|---|---|---|
| Active matter: | | |
| Paracetamol methionate | 428 | 55 |
| Paracetamol | 400 | 50 |
| (Sub-Total of Active Matter) | (828) | (105) |
| Tabletting aids: | | |
| Sodium starch glycolate | 100 | — |
| Magnesium stearate | 8 | — |
| Polyvinylpyrrolidone | 50 | 50 |
| Sodium lauryl sulphate | 2 | — |
| (Sub-Total of Tabletting Aids) | (160) | (50) |
| Totals: | 988 | 155 |
| Granulating Fluid: | Methanol: 300 mls | 237.7 |

Stage B$^1$: Preparation of comparative granulate by conventional moist granulation

A preliminary run, using the above-listed ingredients (excepting 50 grams of the sodium starch glycolate, 8 grams of magnesium stearate and 2 grams of sodium lauryl sulphate) in a standard

mixer-granulator established that approximately 300 mls of methanol (containing the polyvinylpyrrolidone) added as granulating fluid in the conventional manner were needed to obtain the desired moist, coherent, non-pasty mass suitable for granulation.

The mass was then granulated by screening and drying in a fully conventional manner.

Stage $B^2$: Preparation of granulate in accordance with this invention

Using a standard mixer-granulator, the above-listed ingredients were brought together in the following manner:

Preparation of the slurry

The polyvinylpyrrolidone (50 grams) was dissolved in the full amount of methanol predetermined in Stage $B^1$ above, namely 300 mls.

Dissolution was carried out at room temperature under flame-proof conditions, since the flashpoint of methanol is only 54°F (about 13°C).

Under the same conditions, a proportion of both the paracetamol methionate (i.e. 55 grams) and the paracetamol (i.e. 50 grams) was suspended in the PVP-containing methanol by means of a high-sheer homogenizer, to form a stable dispersion having a cream-like consistency, which was used in the manner described below as a granulating slurry in the formation of granulate.

Preparation of the moist mass

The remainder of the paracetamol methionate (i.e. 373 grams) was mixed with the remainder of the paracetamol (350 grams) and half of the sodium starch glycolate (50 grams) in a conventional mixer-granulator, and this dry mixture was wet-granulated (under flame-proof conditions) until suitably massed in a conventional manner except that as the granulating fluid there was used the granulating slurry prepared as described above.

Preparation of the granules

The resultant moist, coherent, non-pasty mass was screened through a 4-mesh sieve, and dried in a fluidized-bed air-drier at a temperature of about 40°C until the odour of methanol had disappeared.

The resultant dried granules were re-screened through a 16-mesh sieve to eliminate agglomerates. They were then blended with the remainder of the sodium starch glycolate (50 grams), with the sodium lauryl sulphate (2 grams) and with the magnesium stearate (8 grams) so as to form the final granulate ready for tabletting.

Stage $C^1$: Preparation of comparative tablets

The granulate prepared as described in Stage $B^1$ above was compressed into approximately 988 mg tablets upon a conventional rotary tabletting machine. The resultant tablets were however unsatisfactory, due to flow and lubrication problems with the granulate, and to lamination of the tablets.

Stage $C^2$: Preparation of tablets according to this invention

The free-flowing tabletting granulate prepared as described in Stage $B^2$ above was fed into the same rotary tabletting machine. The granulate was compacted thereby into tablets which had an overall weight of approximately 988 mg each, and which were found (despite the fact that they contained only approximately 16% of tabletting aids) to conform to accepted standards of appearance, hardness, fragility (friability), disintegration and uniformity of weight.

Typical results which may be obtained using procedures and compositions as described above for Examples 1 and 3 are as follows:

TABLE A

| Type of composition | Appearance | Hardness (kp) | Disintegration time (minutes) | Friability |
|---|---|---|---|---|
| Example 1 | Satisfactory | 11 to 13 | 11 to 13 | Satisfactory i.e. less than 1% |
| Example 1 | " | 8 | 5 | " |
| Example 1 | " | 15 | 12.5 | " |
| Example 3 | " | 14 to 15 | 4 to 10 | " |

Example 4

Preparation of tablets containing benorylate using either ethanol or water as the granulating fluid in the granulating slurry

Stage A: Preparation of tabletting mixture

A tabletting mixture was formed by mixing together the ingredients which are listed in Table IV below, in the absolute and proportionate amounts also shown in Table IV, by means of the procedure described subsequently herein:

TABLE IV

| Ingredients | Quantity in whole mixture (grams) | Amount in slurry (grams) |
|---|---|---|
| Active matter: | | |
| Benorylate | 750 | 250 |
| (Sub-Total of Active Matter) | (750) | (250) |
| Tabletting aids: | | |
| Polyvinylpyrrolidone | 18.75 | 18.75 |
| Potassium sorbate | 0.75 | 0.75 |
| Maize starch | 23 | — |
| Sodium starch glycolate | 21.75 | — |
| Magnesium stearate | 3.75 | — |
| (Sub-Total of Tabletting Aids) | (68) | (19.5) |
| Total: | 818 | 269.5 |
| Granulating Fluid: | Ethanol: 350 mls | 285.6 |

Stage B: Preparation of the granulate

The procedure used for the preparation of the granulate from the above-listed ingredients was as follows:

Preparation of the slurry

The polyvinylpyrrolidone (18.75 grams) and the potassium sorbate (0.75 grams) were dissolved in ethanol (350 ml). One third of the benorylate (250 grams) was then suspended in the ethanolic solution by means of a high-shear homogenizer, to form a stable dispersion having a cream-like consistency, which is used as the granulating slurry in the manner described below.

Preparation of the moist mass

The remainder of the benorylate (500 grams) was mixed with the maize starch (23 grams) in a conventional mixer-granulator. The mixture was then wet-granulated until suitably massed in an otherwise conventional manner except that as granulating fluid there was used the granulating slurry prepared as described above.

Preparation of the granulate

The resultant moist, coherent, non-pasty granules were screened through a 4-mesh sieve, and dried in a fluidized-bed air-drier at a temperature of from 40° to 50°C until the odour of ethanol had disappeared.

The resultant dried granules were re-screened through a 16-mesh sieve to eliminate agglomerates. They were then blended with sodium starch glycolate (21.75 grams) and magnesium stearate (3.75 grams) so as to form the final granulate.

Stage C: Preparation of tablets according to this invention

The free-flowing tabletting granulate, prepared as described in Stage B above, was fed into a conventional rotary tabletting machine, and compressed to form approximately 818 mg caplets. These were found to conform to accepted standards of appearance, hardness, fragility (friability), disintegration and uniformity of weight.

—0—0—0—0—0—

The same procedure utilizing water (350 ml=350 grams) as the granulating fluid instead of ethanol, and drying the granules to a moisture content of less than 2%, yielded equally satisfactory caplets.

Example 5

Preparation of tablets containing nalidixic acid, using isopropanol or ethanol or aqueous (50:50) mixtures thereof as the granulating fluid in the granulating slurry

Stage A: Preparation of tabletting mixture

A tabletting mixture was formed by mixing together the ingredients which are listed in Table V, below, in the absolute and proportionate amounts shown in Table V, by means of the procedure described subsequently herein:

TABLE V

| Ingredients | Quantity in whole mixture (grams) | Amounts in slurry (grams) |
|---|---|---|
| Active matter: | | |
| Nalidixic acid | 500 | 165 |
| (Sub-Total of Active Matter) | (500) | (165) |
| Tabletting aids: | | |
| Polyvinylpyrrolidone | 37 | 37 |
| Maize starch | 138 | — |
| Dicalcium phosphate | 100 | — |
| Magnesium stearate | 2 | — |
| (Sub-Total of Tabletting Aids) | (277) | (37) |
| Totals: | 777 | 202 |
| Granulating Fluid: | *Iso*propanol: 200 mls | 157 |

Stage B: Preparation of the granulate

The procedure used for the preparation of the granulate from the above-listed ingredients was as follows:

Preparation of the slurry

The polyvinylpyrrolidone (37 grams) was dissolved in the *iso*propanol (200 mls=157 grams). About one-third of the nalidixic acid (165 grams) was then suspended in the *iso*propanol solution by means of a high-shear homogenizer, to form a stable dispersion having a cream-like consistency, which was used as the granulating slurry in the manner described below.

Preparation of the moist mass

The remainder of the nalidixic acid (335 grams) was mixed with the maize starch (138 grams) and the dicalcium phosphate (100 grams) in a conventional mixer-granulator. The mixture was then wet-granulated until suitably massed in an otherwise conventional manner except that as the granulating fluid there was used the granulating slurry prepared as described above.

Preparation of the granulate

The resultant moist, coherent, non-pasty granules were screened through a 4-mesh sieve, and dried in a fluidized-bed air-drier at a temperature of from about 50° to 60° until the odour of *iso*propanol could no longer be detected.

The resultant dried granules were re-screened through a 16-mesh sieve to eliminate agglomerates. They were then blended with the magnesium stearate (2 grams) so as to form the final granulate.

Stage C: Preparation of tablets according to this invention

The free-flowing tabletting granulate, prepared as described in Stage B above was fed into a conventional rotary tabletting machine, and compressed to form both approximately 777 mg tablets and approximately 777 mg caplets. These were found to conform to accepted standards of appearance, hardness, fragility (friability), disintegration and uniformity of weight.

—0—0—0—0—0—

The same procedure yielded equally satisfactory tablets and caplets when instead of *iso*propanol there was employed as the granulating fluid the same volume of ethanol (156 grams) or of aqueous (50:50) *iso*propanol (178.5 grams) or of aqueous (50:50) ethanol (178 grams).

Example 6

Preparation of tablets containing hexopal using ethanol as the granulating fluid in the granulating slurry

A tabletting mixture was formed by mixing together the ingredients which are listed in Table VI below, in the absolute and proportionate amounts also shown in Table VI, by means of the procedure described subsequently herein:

TABLE VI

| Ingredients | Quantity in whole mixture (grams) | Amounts in slurry (grams) |
|---|---|---|
| Active Matter: | | |
| *meso*-Inositol hexanicotinate | 1000 | 300 |
| (Sub-Total of Active Matter) | (1000) | (300) |
| Tabletting aids: | | |
| Polyvinylpyrrolidone | 50 | 50 |
| Maize starch | 75 | — |
| Sodium lauryl sulphate | 10 | — |
| Magnesium stearate | 8 | — |
| (Sub-Total of Tabletting Aids) | (143) | (50) |
| Totals: | 1143 | 350 |
| Granulating Fluid: | Ethanol: 300 mls | 240 |

Stage B: Preparation of the granulate

The procedure used for the preparation of the granulate from the above-listed ingredients was as follows:

Preparation of the slurry

The polyvinylpyrrolidone (50 grams) was dissolved in the ethanol (300 ml=240 grams). Roughly a third of the *meso*-inositol hexanicotinate (300 grams) was then suspended in the ethanolic solution by means of a high-shear homogenizer, to form a stable dispersion having a cream-like consistency, which was used as the granulating slurry in the manner described below.

Preparation of the moist mass

The remainder of the *meso*-inositol hexanicotinate (700 grams) was then placed in a conventional mixer-granulator and wet-granulated until suitably massed in an otherwise conventional manner except that as the granulating fluid there was used the slurry prepared as described above.

Preparation of the granulate

The resultant moist, coherent, non-pasty granules were screened through a 4-mesh sieve, and dried in a fluidized-bed air-drier at a temperature of from about 50° to 60°C until the odour of ethanol could no longer be detected.

The resultant dried granules were re-screened through a 16-mesh sieve to eliminate agglomerates. They were then blended with the maize starch (75 grams), the sodium lauryl sulphate (10 grams) and the magnesium stearate (8 grams) so as to form the final granulate.

Stage C: Preparation of tablets according to this invention

The free-flowing tabletting granulate, prepared as described in Stage B above, was fed into a conventional rotary tabletting machine, and compressed to form approximately 1143 mg caplets. These were found to conform to accepted standards of appearance, hardness, fragility (friability), disintegration and uniformity of weight.

—0—0—0—0—0—

Example 7

Alternative preparation of tablets containing both paracetamol and paracetamol methionate using methanol as the granulating fluid

Stage A: Preparation of tabletting mixture

A tabletting mixture was formed by mixing together the ingredients which are listed in Table VII below, in the absolute and proportionate amounts shown in Table VII, by means of the procedure described subsequently herein:

TABLE VII

| Ingredients | Quantity in whole mixture (grams) | Amount in slurry (grams) |
|---|---|---|
| Active matter: | | |
| Paracetamol methionate | 428 | 55 |
| Paracetamol | 400 | 50 |
| (Sub-Total of Active Matter) | (828) | (105) |
| Tabletting aids: | | |
| Sodium starch glycolate | 100 | — |
| Magnesium stearate | 8 | — |
| Polyvinylpyrrolidone | 50 | — |
| Sodium lauryl sulphate | 2 | — |
| (Sub-Total of Tabletting Aids) | (160) | (NIL) |
| Totals: | 988 | 105 |
| Granulating Fluid: | Methanol: 300 mls | 237.7 |

Stage B$^1$: Preparation of comparative granulate by conventional moist granulation

A preliminary run, using the above-listed ingredients (excepting 50 grams of the sodium starch glycollate, 8 grams of magnesium stearate and 2 grams of sodium lauryl sulphate) in a standard mixer-granulator established that approximately 300 mls of methanol added as granulating fluid in the conventional manner were needed to obtain the desired moist, coherent, non-pasty mass suitable for granulation.

The mass was then granulated by screening and drying in a fully conventional manner.

Stage $B^2$: Preparation of granulate in accordance with this invention

Using a standard mixer-granulator, the above-listed ingredients were brought together in the following manner:

Preparation of the slurry

Working under flame-proof conditions at room temperature, since the flashpoint of methanol is only 54°F (about 13°C), a proportion of both the paracetamol methionate (i.e. 55 grams) and the paracetamol (i.e. 50 grams) was suspended in the full amount of methanol predetermined in Stage $B^1$ above, namely 300 mls, by means of a high-shear homogenizer, to form a stable dispersion having a cream-like consistency. That dispersion was then used in the manner described below as a granulating slurry in the formation of granulate.

Preparation of the moist mass

The remainder of the paracetamol methionate (i.e. 373 grams) was mixed with the remainder of the paracetamol (350 grams), half of the sodium starch glycolate (50 grams) and all the polyvinylpyrrolidone (50 grams) in a conventional mixer-granulator, and this dry mixture was wet-granulated (under flame-proof conditions) until suitably massed in a conventional manner except that as the granulating fluid there was used the granulating slurry prepared as described above.

Preparation of the granules

The resultant moist, coherent, non-pasty mass was screened through a 4-mesh sieve, and dried in a fluidized-bed air-drier at a temperature of about 40°C until the odour of methanol had disappeared. The resultant dried granules were re-screened through a 16-mesh sieve to eliminate agglomerates; and they were then blended with the remainder of the sodium starch glycollate (50 grams), with the sodium lauryl sulphate (2 grams) and with the magnesium stearate (8 grams) so as to form the final granulate ready for tabletting.

Stage $C^1$: Preparation of comparative tablets

The granulate prepared as described in Stage $B^1$ above was compressed into approximately 988 mg tablets upon a conventional rotary tabletting machine. The resultant tablets were however unsatisfactory, due to flow and lubrication problems with the granulate and to lamination of the tablets.

Stage $C^2$: Preparation of tablets according to this invention

The free-flowing tabletting granulate prepared as described in Stage $B^2$ above was fed into the same rotary tabletting machine. It was then compacted thereby into tablets which had an overall weight of approximately 988 mg, and which were found (despite the fact that they contained only approximately 16% of tabletting aids) to conform to accepted standards of appearance, hardness, fragility (friability), disintegration and uniformity of weight.

—0—0—0—0—0—0—

Example 8

Preparation of tablets containing paracetamol using water as the granulating fluid in the granulating slurry

A tabletting mixture was formed by mixing together the ingredients which are listed in Table VIII below, in the absolute and proportionate amounts also shown in Table VIII, by means of the procedure described subsequently herein:

TABLE VIII

| Ingredients | Quantity in whole mixture (grams) | Amounts in slurry (grams) |
|---|---|---|
| Active matter: Paracetamol | 500 | 150 |
| (Sub-Total of Active Matter) | (500) | (150) |
| Tabletting aids: Polyvinylpyrrolidone | 3 | 3 |
| Sorbitol powder | 100 | 75 |
| Carbowax (Trade Mark) 6000 | 12 | 12 |
| Stearic acid | 4 | — |
| Talc | 20 | — |
| Maize starch | 17 | — |
| Soluble starch | 35 | — |
| (Sub-Total of Tabletting aids) | (191) | (90) |
| Totals: | 691 | 240 |
| Granulating Fluid: | Water: 100 mls | 100 |

[Note: Carbowax 6000 is believed to be a mixture of polyethylene glycols and methoxypolyethylene glycols having an average molecular weight of approximately 6,000].

Stage B: Preparation of the granulate

The procedure used for the preparation of the granulate from the above-listed ingredients was as follows:

Preparation of the slurry

The polyvinylpyrrolidone (3 grams) and three-quarters of the sorbitol powder (75 grams) were dissolved in water (100 ml=100 grams). Roughly a third of the paracetamol (150 grams) and the Carbowax® 6000 (12 grams) were then suspended in the aqueous solution by means of a high-shear homogenizer, to form a stable dispersion having a cream-like consistency, which was used as the granulating slurry in the manner described below.

Preparation of the moist mass

The remainder of the paracetamol (350 grams) was mixed with the soluble starch (35 grams) and the rest of the sorbitol powder (25 grams) in a conventional mixer-granulator. The mixture was then wet-granulated until suitably massed in an otherwise conventional manner except that as the granulating fluid there was used the granulating slurry prepared as described above.

Preparation of the granulate

The resultant moist, coherent, non-pasty granules were screened through a 4-mesh sieve, and dried in a fluidized-bed air-drier at a temperature of from 50° to 60°C until the moisture level had droped below 2% w/w.

The resultant dried granules were re-screened through a 16-mesh sieve to eliminate agglomerates. They were then blended with the talc (20 grams), stearic acid (4 grams) and maize starch (17 grams) so as to form the final granulate.

Stage C: Preparation of tablets according to this invention

The free-flowing tabletting granulate, prepared as described in Stage B above, was fed into a

conventional rotary tabletting machine, and compressed to form one-half inch diameter tablets. These were found to conform to accepted standards of appearance, hardness, fragility (friability), disintegration and uniformity of weight.

—0—0—0—0—0—0—

Example 9
Preparation of caplets containing paracetamol and DL-methionine using wholly-aqueous granulating slurry

Stage A: Preparation of tabletting mixture
A tabletting mixture was formed by mixing together the ingredients which are listed in Table IX below, in the absolute and proportionate amounts also shown in Table IX, by means of the procedure described subsequently herein:

TABLE IX

| Ingredients | Total quantity in grams | Amount in slurry |
|---|---|---|
| Active-Matter | | |
| DL-Methionine | 250 | 125 |
| Paracetamol | 500 | — |
| (Sub-Total of Active Matter) | (750) | (125) |
| Tabletting aids | | |
| Polyvinylpyrrolidone | 30 | 30 |
| Sodium acetate | 13 | 13 |
| Stearic acid | 10 | — |
| Sodium Starch Glycolate | 50 | — |
| (Sub-Total of Tabletting Aids) | (68) | (43) |
| Total | 853 | 168 |
| Granulating Fluid | Water:85 mls | 85 grams |

Stage B: Preparation of the granulate
The procedure used for the preparation of the granulate from the above-listed ingredients was as follows:

Preparation of the slurry
The sodium acetate (13 grams) was dissolved in 85 mls of water and the polyvinylpyrrolidone (30 grams) was dissolved in the aqueous solution of sodium acetate. Half of the DL-methionine (i.e. 125 grams) was suspended in the thus-formed aqueous solution by means of a high-shear homogenizer to form a stable dispersion having a cream-like consistency, which was used as the granulating slurry in the manner described below.

Preparation of the moist mass
The remainder of the DL-methionine (i.e. 125 grams) was mixed with the paracetamol (500 grams) and half the sodium starch glycolate (25 grams) in a conventional mixer-granulator. The mixture was then wet-granulated until suitably massed in an otherwise conventional manner except that as granulating fluid there was used the granulating slurry prepared as described in Stage A above. (If required a small amount of additional water may be added to achieve the moist-mass condition).

Preparation of the granules
The resultant moist, coherent, non-pasty granulate was screened through a 4-mesh sieve, and dried in a fluidized-bed drier at a temperature of about 60°C until a moisture-content of 1.5% to 2% w/w had been achieved.
The resulting dried granules were re-screened through a 16-mesh sieve to eliminate agglomerates and

then blended with stearic acid (10 grams) and the remainder of the sodium starch glycollate (25 grams) to form the final granulate ready for tabletting.

Stage C: Preparation of tablets

The free-flowing tabletting granulate prepared as described in Stage B above was fed into a suitable conventional tablet-press fitted with caplet tooling, and compacted thereby into caplets which had an overall weight of approximately 853 mg each. Despite the fact that paracetamol is poorly-compressible and DL-methionine still more so, yet these were together present to an extent of nearly 88% w/w, with only about 12% of tabletting aids and no mere bulking agents. Furthermore, these caplets were found to conform to accepted standards of appearance, hardness, fragility (friability), disintegration and uniformity of weight as can be seen for three different batches from the results given in Table B below:

TABLE B

| Batch | Uniformity of weight | Appearance | Hardness (kp) | Disintegration time (minutes) | Fragility (friability) |
|---|---|---|---|---|---|
| A | Satisfactory ±5% | Satisfactory | 14.7 | Satisfactory— less than 15 minutes* | Satisfactory— 0.05% weight loss |
| B | " | " | 8.7 | " | Satisfactory— 0.12% weight loss |
| C | " | " | 12.7 | " | Satisfactory— 0.12% weight loss |

*British Pharmacopoeia Standard BP 1980

Example 10

Preparation of tablets containing both paracetamol and paracetamol methionate using water as the granulating fluid

Stage A: Preparation of tabletting mixture

A tabletting mixture was formed by mixing together the ingredients which are listed in Table X below, in the absolute and proportionate amounts also shown in Table X, by means of the procedure described subsequently herein.

TABLE X

| Ingredients | Quantity in whole mixture (grams) | Amount in slurry (grams) |
|---|---|---|
| Active matter: | | |
| Paracetamol methionate | 428 | 42.8 |
| Paracetamol | 400 | 40 |
| (Sub-Total of Active Matter) | (828) | (82.8) |
| Tabletting aids: | | |
| Sodium starch glycolate | 100 | — |
| Magnesium stearate | 8 | — |
| Polyvinylpyrrolidone | 40 | 40 |
| Sodium lauryl sulphate | 10 | 10 |
| (Sub-Total of Tabletting Aids) | (158) | (50) |
| Totals: | 986 | 132.8 |
| Granulating Fluid: | Water: 310 mls | 310 grams |

Stage B[1]: Preparation of comparative granulate by conventional moist granulation

A preliminary run, using the above-listed ingredients (excepting 50 grams of the sodium starch glycolate and 8 grams of magnesium stearate) in a standard mixer-granulator established that approximately 310 mls of water (containing the polyvinylpyrrolidone and sodium lauryl sulphate) added as granulating fluid in the conventional manner were needed to obtain the desired moist, coherent, non-pasty mass suitable for granulation.

The mass was then granulated by screening and drying in a fully conventional manner.

Stage B[2]: Preparation of granulate in accordance with this invention

Using a standard mixer-granulator, the above-listed ingredients were brought together in the following manner:

Preparation of the slurry

The polyvinylpyrrolidone (40 grams) and sodium lauryl sulphate (10 grams) were dissolved in the full amount of water predetermined in Stage B[1] above, namely 310 mls. Dissolution was carried out at room temperature and under the same conditions, a proportion of both the paracetamol methionate (i.e. 42.8 grams) and the paracetamol (i.e. 40 grams) was suspended in the PVP- and sodium lauryl sulphate-containing water by means of a high-shear homogenizer, to form a stable dispersion having a cream-like consistency, which was used in the manner described below as a granulating slurry in the formation of granulate.

Preparation of the moist mass

The remainder of the paracetamol methionate (i.e. 385.1 grams) was mixed with the remainder of the paracetamol (360 grams) and half of the sodium starch glycolate (50 grams) in a conventional mixer-granulator, and this dry mixture was wet-granulated until suitably massed in a conventional manner except that as the granulating fluid there was used the granulating slurry prepared as described above.

Preparation of the granules

The resultant moist, coherent, non-pasty mass was screened through a 4-mesh sieve, and dried in a fluidized-bed air-drier at a temperature of about 55°C to a moisture content of about 1.5% w/w.

The resultant dried granules were re-screened through a 16-mesh sieve to eliminate agglomerates. They were then blended with the remainder of the sodium starch glycolate (50 grams) and with the magnesium stearate (8 grams) so as to form the final granulate ready for tabletting.

Stage C[1]: Preparation of comparative tablets

The granulate prepared as described in Stage B[1] above was compressed into approximately 986 mg tablets upon a conventional rotary tabletting machine. The resultant tablets were however unsatisfactory, due to flow and lubrication problems with the granulate, and to lamination of the tablets.

Stage C[2]: Preparation of tablets according to this invention

The free-flowing tabletting granulate prepared as described in Stage B[2] above was fed into the same rotary tabletting machine. The granulate was compacted thereby into tablets which had an overall weight of approximately 986 mg each, and which were found (despite the fact that they contained only approximately 16% of tabletting aids) to conform to accepted standards of appearance, hardness, fragility, (friability) disintegration and uniformity of weight.

**Claims**

1. A process for preparing a granulate suitable for use in manufacturing tablets by compressing and compacting a said granulate, which process comprises forming an intimate mixture of particulate-solid materials including active matter, for example, one or more pharmacologically-active substances, and tabletting aids, as well as optionally other pharmacologically-inert material(s), thereafter "wet-granulating" said mixture by moistening it with granulating fluid(s) to form a substantially-uniform, moist, coherent, non-pasty mass which finally is subdivided into individual granules and dried, the dried granules (optionally after adding further tabletting aids thereto) constituting the desired granulate ready for compression and compaction into tablets, the process being characterised by including the steps of:

(A) predetermining a quantity of granulating fluid(s) needed to convert the entire particulate-solid mixture into a desired moist, coherent, non-pasty mass;

(B) homogenizing part only of the particulate-solid material(s), separately from the remainder thereof, with a chosen amount of granulating fluid(s), that chosen amount being at least 90%, and preferably 100%, by weight of the aforesaid predetermined quantity of granulating fluid(s), so as to form a substantially-homogeneous slurry wherein the percentage by weight of solids in the slurry, namely:

$$\frac{\text{Total Solids, i.e. both dissolved and undissolved} \times 100}{\text{Total Slurry, i.e. fluids plus total solids}}$$

is at least about 25% w/w; and

(C) moistening the remaining part of the particulate-solid material(s) in the manner of "wet-granulation" but with the slurry resultant from step (B) above (and thereafter as necessary with the balance of the granulating fluid(s), if any) so as thus to form desired substantially-uniform, moist, coherent, non-pasty mass ready for granulation.

2. A process according to claim 1, in which the homogenization into the form of a slurry is performed by means of a high-shear homogenizer.

3. A process according to claim 1 or claim 2, in which that part of the particulate-solid materials which is homogenized with the chosen amount of granulating fluid(s) is or includes some or all of the active-matter.

4. A process according to claim 3, in which that part of the particulate-solid materials which is homogenized with the chosen amount of granulating fluid(s) consists of or includes not more than 50% and not less than 10% by weight, and is preferably from 15% to 35% by weight, of the total active matter.

5. A process according to any of the preceding claims, in which that part of the particulate-solid materials which is homogenized with the chosen amount of granulating fluid(s) does not exceed 50% but is not less than 10% by weight, and is preferably from 15% to 35% by weight, of the total particulate-solid materials.

6. A process according to any of the preceding claims, in which that part of the particulate-solid materials which is homogenized with the chosen amount of granulating fluid(s) is or includes poorly-compressible active matter.

7. A process according to any of the preceding claims, in which the particulate-solid materials contain 80% w/w or more of active-matter.

8. A process according to claim 6 and/or claim 7, in which the total tabletting aid(s) incorporated in the granulate before compression into tablets amount to from 5% to 25% w/w, preferably from 5% to 15% w/w, overall calculated relative to the weight of the final dry mass and are, for example, some or all of the following:

binders in the range of from 2% to 10% w/w;
glidants in the range of from 0.2% to 10% w/w;
lubricants in the range of from 0.2% to 4% w/w; and
disintegrants in the range of from 2% to 10% w/w.

9. A process according to claim 6 and/or 7 and/or 8, in which the active matter homogenized is or includes one of the following, namely nalidixic acid, paracetamol with or without methionine, hexopal, benorylate and paracetamol methionate with or without paracetamol.

10. A process as claimed in any of the preceding claims, in which the percentage by weight of solids in the slurry does not exceed 80% w/w, preferably does not exceed 70% w/w, and more preferably is from 40% w/w up to 60% w/w, e.g. ±50% w/w.

11. A process according to any of the preceding claims, in which the tabletting aids employed include a binding agent, for example, polyvinylpyrrolidone, at least part of which is dissolved in the granulating fluid(s) incorporated in the slurry.

12. A process according to any of the preceding claims, in which the granulating fluid(s) employed to form the granulating slurry consist of or include water and/or one or more substantially non-toxic, organic liquid(s) more volatile than water, for example, water and/or one or more water-miscible relatively-volatile polar organic liquids such as methanol, ethanol, isopropanol, acetone and/or methyl ethyl ketone; or one or more water immiscible relatively-volatile organic liquids, for example, methylene chloride, chloroform and/or ether.

13. A process according to any of the preceding claims, in which after the remaining part of the particulate-solid material(s) has been uniformly moistened by means of the granulating slurry, the resultant moist, coherent, non-pasty mass is broken down into moist granules by screening.

14. The use of a granulate prepared by a process according to any of the preceding claims for the manufacture of tablets by compression and compaction thereof.

15. A process according to any of claims 1 to 13, which includes the further step, optionally after blending the dried granules with one or more further tabletting aids, of compacting the resultant granulate to form tablets of a predetermined tablet volume in tabletting machinery.

16. Tablets whenever manufactured by a process according to claim 15.

17. A tablet of overall weight no more than 1 g, which tablet comprises a unit dose of active matter of at least 700 mg, the active matter being paracetamol together with methionine or paracetamol methionate, or paracetamol methionate alone, present in an amount of from 80 to 90% by weight, the tablet having a hardness of from 7 to 15 kp, a friability of less than 2%, preferably less than 1%, and affording a disintegration time of no more than 15 minutes, preferably from 5 to 15 minutes.

**Patentansprüche**

1. Ein Verfahren zur Herstellung eines Granulats, das zur Produktion von Tabletten eingesetzt werden kann, indem besagtes Granulat komprimiert und verdichtet wird, wobei das Verfahren die Bildung einer engen Mischung von festen Partikeln des Materials einschliesslich aktiver Substanzen umfasst, beispielsweise eine oder mehrere pharmakologisch aktive Substanzen, sowie Tablettierhilfsstoffe und wahlweise anderes pharmakologisch inertes Material oder Materialien, anschliessendes "Nassgranulieren" besagter Mischung, indem diese mit Granulierflüssigkeit(en) angefeuchtet wird, um eine hochgradig einheitliche, feuchte, kohärente, nicht breiige Masse zu formen, die schliesslich in einzelne Granule aufgeteilt und getrocknet wird, so dass die getrockneten Granulate (nach wahlweisem Zusatz von weiteren Tablettierhilfsstoffen) die gewünschten Granulate darstellen und durch Komprimieren und Verdichten zu Tabletten geformt werden können, wobei das Verfahren durch folgende Schritte gekennzeichnet ist:

A) Vorgängige Bestimmung der benötigten Menge an Granulierflüssigkeit(en) zur Bildung der gewünschten, feuchten, kohärenten, nicht breiigen Masse aus der gesamten Mischung fester Partikel;

B) Homogenisierung eines Teils der Mischung aus festen Partikeln, getrennt vom Rest besagter Mischung, mittels einer bestimmten Menge an Granulierflüssigkeit(en), wobei besagte menge an Granulierflüssigkeit(en) wenigstens 90% und idealerweise 100% des Gewichts besagter, vorgängig bestimmter Menge an Granulierflüssigkeit(en) ausmacht/ausmachen, so dass eine hochgradig homogene breiige Masse hergestellt werden kann, in welcher der prozentuale Gewichtsanteil der festen Partikel, nämlich:

$$\frac{\text{Festkörper total, das heisst gelöste und ungelöste Festkörper} \times 100}{\text{Brei total, das heisst Total der Flüssigkeit und Festkörper}}$$

wenigstens 25% ausmacht; und

C) Anfeuchten des Rests des/der Material(ien) aus festen Partikeln durch "Nassgranulieren", aber mit der breiigen Masse aus vorangehendem Schritt B (und, falls erforderlich, nachfolgendem Ausgleich der Granulierflüssigkeit(en), so vorhanden, um die gewünschte, hochgradig einheitliche, feuchte, kohärente, nicht breiige Masse zur Granulierung herzustellen.

2. Ein Verfahren nach Anspruch 1, dadurch gekennzeichnet, dass die Homogenisierung in eine breiige Masse mittels eines Homogenisierapparates auf der Basis des Zerkleinerungsprinzips erfolgt.

3. Ein Verfahren nach Anspruch 1 oder 2, dadurch gekennzeichnet, dass der Teil des Materials aus festen Partikeln, welches unter Beigabe einer bestimmten Menge an Granulierflüssigkeit(en) homogenisiert wird, eine aktive Substanz ist oder Teile oder die gesamte aktive Substanz enthält.

4. Ein Verfahren nach Anspruch 3, dadurch gekennzeichnet, dass der Teil des Materials aus festen Partikeln, welches unter Beigabe einer bestimmten Menge an Granulierflüssigkeit(en) homogenisiert wird, aus nicht mehr als 50% und nicht weniger als 10% und aus vorzugsweise zwischen 15% und 35% der gesamten aktiven Substanz besteht oder diese in besagten prozentualen Gewichtsanteilen enthält.

5. Ein Verfahren nach einem der vorangehenden Ansprüche, dadurch gekennzeichnet, dass der Teil des Materials aus festen Partikeln, welches unter Beigabe einer bestimmten Menge an Granulierflüssigkeit(en) homogenisiert wird, einen Anteil von 50% am Gesamtgewicht des Materials an festen Partikeln nicht überschreitet, aber nicht weniger als 10% ausmacht und vorzugsweise zwischen 15% und 35% liegt.

6. Ein Verfahren nach einem der vorangehenden Ansprüche, dadurch gekennzeichnet, dass der Teil des Materials aus festen Partikeln, welches unter Beigabe einer bestimmten Menge an Granulierflüssigkeit(en) homogenisiert wird, aus schlecht komprimierbarem Material besteht oder solches enthält.

7. Ein Verfahren nach einem der vorangehenden Ansprüche, dadurch gekennzeichnet, dass das Material aus festen Partikeln einen Gewichtsanteil von 80% oder mehr an aktivem Material enthält.

8. Ein Verfahren nach Anspruch 6 und/oder 7, dadurch gekennzeichnet, dass der gesamte Tablettierhilfsstoff oder die gesamten Tablettierhilfsstoffe im Granulat vor der Komprimierung in Tabletten einen Gewichtsanteil zwischen 5% und 20%, vorzugsweise zwischen 5% und 15% in Relation zum Gewicht der trockenen Masse nach Abschluss des Verfahrens ausmacht/ausmachen und sich, beispielsweise, aus einigen oder allen der folgenden komponenten zusammensetzt/zusammensetzen:

Bindemittel mit einem Gewichtsanteil zwischen 2% und 10%;
Gleitmittel (glidants) mit einem Gewichtsanteil zwischen 0.2% und 10%;
Gleitmittel (lubricants) mit einem Gewichtsanteil zwischen 0,2% und 4%; und
Aufschlussmittel mit einem Gewichtsanteil zwischen 2% und 10%.

9. Ein Verfahren nach Anspruch 6 und/oder 7 und/oder 8, dadurch gekennzeichnet, dass das homogenisierte aktive Material aus einer der folgenden Komponenten besteht oder diese enthält: Naladixinsäure, Paracetamol mit oder ohne Methionin, Hexopal, Benorilat und Paracetamolmethionat mit oder ohne Paracetamol.

10. Ein Verfahren nach einem der vorangehenden Ansprüche, dadurch gekennzeichnet, dass der

prozentuale Gewichtsanteil der Festkörper in der breiigen Masse 80% nicht überschreitet, vorzugsweise 70% nicht überschreitet und sich idealerweise zwischen 40% und 60% bewegt, das heisst +/− 50% 5% Gewichtsanteil (sic.).

11. Ein Verfahren nach einem der vorangehenden Ansprüche, dadurch gekennzeichnet, dass die benutzten Tablettierhilfsstoffe ein Bindemittel enthälten, beispielsweise Polyvinylpyrrolidon, wobei wenigsten ein Teil des Bindemittels in der/den in der breiigen Masse enthaltenen Granulierflüssigkeit(en) gelöst ist.

12. Ein Verfahren nach einem der vorangehenden Ansprüche, dadurch gekennzeichnet, dass die Granulierflüssigkeit(en) zur Bildung des Granulierbreis aus Wasser besteht oder enthält und/oder eine(r) oder mehrere(r) nicht toxischer, organische(r) Flüssigkeit(en), die volatiler sind als Wasser, beispielsweise Wasser und/oder mit Wasser mischbaren, relativ volatilen, polaren organischen Flüssigkeiten, wie Methanol, Ethanol, Isopropanol, Aceton, und/oder Methylethylketon; oder eine(r) oder mehrere(n) mit Wasser unvermischbaren, relativ volatilen, organischen Flüssigkeiten, beispielsweise Methylenchlorid, Chloroform und/oder Aether.

13. Ein Verfahren nach einem der vorangehenden Ansprüche, dadurch gekennzeichnet, dass der Rest des Materials oder der Materialien aus festen Partikeln durch den Granulierbrei gleichmässig angefeuchtet worden ist und die dadurch entstandene feuchte, kohärente, nicht breiige Masse mittels Sieben oder Trennen in feuchte Granule verarbeitet wird.

14. Die Anwendung eines Granulats zur Herstellung von Tabletten mittels Komprimieren oder Verdichten des besagten Granulats, wobei besagtes Granulat nach einem in den vorangehenden Ansprüchen beschriebenen Verfahren hergestellt worden ist.

15. Ein Verfahren nach einem der Ansprüche 1 bis 13, dadurch gekennzeichnet, dass weitere Schritte folgen, nachdem die getrockneten Grnaule mit einem oder mehreren weiteren Tablettierhilfsstoff(en) gemischt worden sind, wobei diese Schritte im Verdichten der so erhaltenen Granule zur Herstellung von Tabletten mit einem vorbestimmten Volumen in einer Tablettenmaschine bestehen.

16. Tabletten, die nach einem in Anspruch 15 beschriebenen Verfahren hergestellt worden sind.

17. Eine Tablette mit einem Gesamtgewicht von nicht mehr als 1 g, wobei besagte Tablette eine Dosis aktives Material von wenigstens 700 mg enthält und besagtes Material Paracetamol zusammen mit Methionin oder Paracetamolmethionin oder ausschliesslich Paracetamolmethionin ist mit einem Gewichtsanteil zwischen 80% und 90% und die Tablette eine Härte zwischen 7 und 15 kp aufweist sowie sich in einer Zeit von nicht mehr als 15 Minuten, vorzugsweise zwischen 5 und 15 Minuten, auflöst.

**Revendications**

1. Un procédé pour la préparation d'un granulé destiné à la fabrication, par compression et compactage dudit granulé, de médicaments sous forme de comprimés, procédé comprenant le mélange intime de particules de matières solides incluant un principe actif, par exemple, une ou plusieurs substances pharmacologiquement actives et des excipients pour comprimés et inclut également en option, une ou plusieurs substances pharmacologiquement inertes(s), procédé dans lequel la "granulation humide" s'effectue par un mouillage du mélange à l'aide d'un ou de plusieurs fluides de granulation pour former une masse non-pâteuse, humide, consistante et substantiellement uniforme que l'on divise alors et que l'on sèche enfin en granulés individuels, les granulés secs (éventuellement après addition d'autres excipients pour comprimés) constituant le granulé voulu, prêt à la compression et au compactage, procédé que caractérisent les phases suivantes:

(A) évaluation de la quantité du ou des fluides de granulation permettant de transformer le mélange de particules solides en une masse humide, consistante et non pâteuse.

(B) Une quantité choisie de fluide(s) de granulation pour homogénéiser uniquement une part de le ou des particules de matière(s) solide(s), séparée de reste de cette ou de ces dernières, cette quantité choisie étant au moins de 90% et de préférence de 100% par poids de la quantité prédéterminée de fluides de granulation indiquée précédemment, pour former une pâte substantiellement homogéne, pâte dans laquelle le pourcentage en solide par poids et principalememt:

$$\text{Total en solide dissous et non dissous} \times 100$$

Le Total de Pâte c'est à dire les fluides plus le total en solide est au minimum de 25% avec ou sans; et

(C) un mouillage de la part restante des matières de particules solides selon le procédé de "granulation humide" à l'exclusion de la pâte issue de la phase (B) ci-dessus (et le cas échéant, mouillage, en tant que de besoin du reste du ou des fluides de granulation.) pour former la masse non-pâteuse voulue, humide, cohérente et substantiellement uniforme prête pour la granulation.

2. Un procédé conforme à la revendication 1 dans lequel un homogénéiseur haute vitesse assure l'homogénéisation en pâte.

3. Un procédé conforme à la revendication 1 ou 2 dans lequel la part de particules de matières solides homogénéisée avec la quantité choisie de fluide(s) de granulation correspond à la totalité ou à une partie de principe actif ou inclut ce dernier.

4. Un procédé en accord avec la revendication 3 dont la part de particules de matières solides

homogénéisée avec la quantité choisie de fluide(s) de granulation, est constituée de, ou inclut une quantité inférieure ou égale à 50% et supérieure ou égale à 10% par poids du principe actif total, et comprise de préférence entre 15 et 35% par poids de ce dernier.

5. Un procédé en accord avec l'une des revendications précédentes dans lequel la part de particules de matières solides homogénéisée avec la quantité choisie de fluide(s) de granulation, est inférieure ou égale à 50% et supérieure ou égale à 10% par poids de particules de matières solides et comprise de préférence entre 15 et 35% par poids de ces dernières.

6. Un procédé en accord avec l'une des revendications précédentes, dans lequel cette part de particules de matières solides homogénéisée avec la quantité choisie de fluide(s) de granulation, constitue ou inclut un principe actif de compressibilité médoicre.

7. Un procédé en accord avec l'une des revendications précédentes dans lequel les particules de matières solides contiennent 80% ou plus de principe actif avec ou sans.

8. Un procédé en accord avec les revendication 6 et/ou 7, dans lequel la quantité d'excipient(s) pour comprimés incorporée au granulé avant compression, est comprise entre 5 et 25% avec ou sans, et de préférence entre 5 et 15% avec ou sans, cette quantité étant globalement calculée en fonction du poids de la masse sèche finale, ces excipients incluant en particulier:

des liants dans une fourchette comprise entre 2% et 10% avec ou sans,
des glissants dans une fourchette comprise entre 0,2% et 10% avec ou sans,
des lubrifiants dans une fourchette comprise entre 0,2% et 4% avec ou sans, et
des désintégrants dans une fourchette comprise entre 2% et 10% avec ou sans.

9. Un procédé conforme à la revendication 6 ou 7 dans lequel le principe actif homogénéisé est ou inclut l'un des produits suivants à savoir acide nalidixique, paracétamol avec ou sans méthionine, hexopal, benorylate et paracétamol méthionate avec ou sans paracétamol.

10. Un procédé en accord avec l'une des revendications précédentes dans lequel le pourcentage de solides par poids dans la pâte est inférieur ou égal à 80% avec ou sans, de préférence inférieur ou égal à 70% avec ou sans et idéalement, compris entre 40% et 60%, soit par exemple 50% +/− 5% avec ou sans.

11. Un procédé en accord avec l'une des revendications précédentes dans lequel les excipients utilisés incluent un liant tel que le polyvinylpyrolidone dont au moins une part est dissoute dans le ou les fluides de granulation incorporés à la pâte.

12. Un procédé en accord avec l'une des revendications précédentes dont le ou les fluides de granulation utilisés à la formation de la pâte de granulation sont de l'eau ou en incluent et/ou sont composés ou incluent on ou plusieurs liquides organiques non toxiques plus volatiles que l'eau, par exemple l'eau et un ou plusieurs liquides polaires relativement volatiles et miscibles dans l'eau tel que le méthanol, l'éthanol, l'isopropanol, l'acétone et/ou l'alcool dénature; un ou plusieurs liquides relativement volatiles et non-miscibles dans l'eau tel que le chlorure de méthylène, le chloroforme et/ou l'eau.

13. Un procédé en accord avec l'une des revendications précédentes dans lequel, après mouillage uniforme à l'aide de la pâte de granulation de la part restante des particules de matières solides, on décompose la masse résiduelle humide cohérente et non pâteuse en granulés par tamisage.

14. L'utilisation d'un granulé préparé selon le procédé conforme à l'une des revendications précédentes, pour fabriquer des médicaments par compression et compactage.

15. Un procédé en accord avec l'une des revendications 1 à 13, incluant une phase supplémentaire, après mélange éventuel des granulés secs à un ou plusieurs excipients, phase dans laquelle on compacte le granulat résultant pour former des comprisés d'un volume prédéterminé dans une machine à fabriquer les comprimés.

16. Sous réserve que le procédé de fabrication de ces comprimés soit conforme à la revendication 15.

17. Des comprimés d'un poids individuel global d'un gramme ou moins, chaque comprimé comprenant une dose de 700 mg minimum de principe actif, ce dernier étant de paracétamol et de la méthionine ou du paracétamol méthionate, ou du paracétamol méthionate présent en quantité de 80 à 90% par poids, la dureté de comprimé étant comprise entre 7 et 15 kp, sa friabilité inférieure à 2% et de préférence inférieure à 1%, son temps de désintégration étant de 15 minutes maximum et de préférence de 5 à 15 minutes.